(19) Europäisches Patentamt
European Patent Office
Office européen des brevets

(11) Veröffentlichungsnummer: **0 337 260 B1**

# (12) EUROPÄISCHE PATENTSCHRIFT

(45) Veröffentlichungstag der Patentschrift: **02.12.92**

(51) Int. Cl.⁵: **C07C 43/315**, C07C 41/48, C07C 47/198, C07C 69/734

(21) Anmeldenummer: **89105921.4**

(22) Anmeldetag: **05.04.89**

## (54) Verfahren zur Herstellung von Enolethern und ihre Verwendung zur Herstellung von Aldehyden.

(30) Priorität: **13.04.88 DE 3812236**

(43) Veröffentlichungstag der Anmeldung:
**18.10.89 Patentblatt 89/42**

(45) Bekanntmachung des Hinweises auf die Patenterteilung:
**02.12.92 Patentblatt 92/49**

(84) Benannte Vertragsstaaten:
**BE CH DE FR GB LI NL**

(56) Entgegenhaltungen:
**EP-A- 0 024 532**
**EP-A- 0 217 089**
**EP-A- 3 705 924**
**DE-A- 3 602 253**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Goetz, Norbert, Dr.**
**Schoefferstrasse 25**
**W-6520 Worms 1(DE)**
Erfinder: **Hupfer, Leopold, Dr.**
**Waltershoehe 3**
**W-6701 Friedelsheim(DE)**
Erfinder: **Himmele, Walter, Dr.**
**Eichenweg 14**
**W-6909 Walldorf(DE)**

EP 0 337 260 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues und verbessertes Verfahren zur Herstellung von Enolethern aus Acetalen oder Ketalen, die ein α-Wasserstoffatom enthalten.

Enolether sind in der Regel sehr wertvolle Bausteine in der organischen Synthese. Sie werden für die Herstellung spezieller Homo- und Copolymerisate eingesetzt und finden Anwendung auf den Gebieten der Lack- und Klebstoffherstellung sowie als Hilfsmittel in der Textil- und Lederindustrie. Weiterhin dienen Enolether als wertvolle Zwischenprodukte für organische Synthesen, z.B. für Diels-Alder-Reaktionen, für die Herstellung von Glutardialdehyden, γ-Pyran und γ-Picolin sowie für die Herstellung von Wirkstoffen, wie Herbiziden, fungiziden, Insektiziden und Pharmazeutika.

Technisch werden Enolether nach Reppe aus Acetylen und Alkoholen in Flüssigphase mit Kaliumhydroxid als Katalysator hergestellt.

Weiterhin lassen sich Enolether durch Alkoholabspaltung von Acetalen herstellen. Dies kann rein thermisch, aber auch homogen- bzw. heterogenkatalysiert erfolgen. Die hierbei eingesetzten Katalysatoren sind teils in ihrer Anwendungsbreite beschränkt, teils in ihrer Aktivität, Selektivität und Standzeit verbesserungsbedürftig.

Auch ist bekannt, daß man Enolether durch Abspaltung von Alkoholen aus Acetalen an Aluminosilikatzeolithen erhalten kann (Proceedings of the 7th Internatiol Zeolite Conference, Poster Papier 2D-13). Diese Zeolithe zeigen in ihrer aciden H-Form Selektivitäten und Umsätze, die noch zu wünschen übrig lassen. Durch einen zusätzlichen Verfahrensschritt einer Na-Dotierung des Zeolithen ist es möglich, die Selektivität bei nicht vollständigem Umsatz zu steigern.

Die Herstellung von Ethylvinylether an einem A-Zeolithen aus Acetaldehyddiethylacetal bekannt. Jedoch zeigt der A-Zeolith geringe Standzeit unter der nötigen Reaktionstemperatur von 350 °C und läßt sich nicht von Koks (Desaktivierung) befreien, da bei den hierfür notwendigen Regeneriertemperaturen von mehr als 500 °C das Kristallgerüst dieses Zeoliths instabil ist.

Aus der US-A-3 705 924 ist ein Verfahren zur Herstellung von Vinylallylethern aus Dialkylacetalen in Gegenwart von $SiO_2$, Molekularsieben, Aluminiumphosphaten, Holzkohle, $Al_2O_3$ und $Na_2HPO_4$ bekannt.

Aus der EP-A 217 089 ist ein Verfahren zur katalystischen Dealkoxylierung von geminalen Dialkoxyverbindungen bekannt.

Der Erfindung lag daher die Aufgabe zugrunde, einen besseren Zugang zu Enolethern zu finden und den Nachteilen der bekannten Verfahren abzuhelfen.

Demgemäß wurde ein neues und verbessertes Verfahren zur Herstellung von Enolethern durch Abspaltung eines Alkohols aus Acetalen oder Ketalen gefunden, welches dadurch gekennzeichnet ist, daß man die Umsetzung in Gegenwart von Phosphorsäure und/oder Hydrogenphosphaten auf einem Trägermaterial und/oder Phoshaten und/oder Zeolithen als Katalysatoren vornimmt.

Die Enolether I sind nach folgender Methode erhältlich:
Die Umsetzung erfolgt durch Kontakt eines Acetals oder Ketals, das ein α-Wasserstoffatom enthält, mit einem Katalysator nach folgender Reaktionsgleichung:

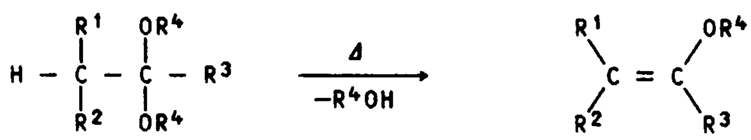


(II) (I)

Die Reaktion kann sowohl in der Flüssigphase als auch in der diskontinuierlichen oder vorzugsweise kontinuierlichen Gasphase bei 50 bis 500 °C und 0,01 bis 50 bar durchgeführt werden.

Die Flüssigphasenreaktion kann beispielsweise als Suspensions-, Riesel-oder Sumpfreaktion bei Temperaturen von 50 bis 200 °C und Drücken von 0,5 bis 20 bar, vorzugsweise bei Temperaturen von 70 bis 170 °C und Drücken von 1 bis 5 bar durchgeführt werden. Es empfiehlt sich, schwerflüchtige oder feste Acetale oder Ketale (II) in einem inerten Lösungsmittel gelöst zu verwenden, wobei pro Mol II zwischen 100 und 500 ml, vorzugsweise 150 bis 350 ml eines inerten Lösungsmittels in aller Regel genügen.

Als inerte Lösungsmittel eignen sich Ether, besonders cyclische Ether wie Tetrahydrofuran und Dioxan; aliphatische Kohlenwasserstoffe wie n-Pentan, das Pentanisomerengemisch, n-Hexan, das Hexanisomerengemisch, Petrolether und Cyclohexan; aromatische Kohlenwasserstoffe wie Benzol, Toluol, die Xylole und

deren Isomerengemisch oder Mischungen dieser Lösungsmittel.

Die bevorzugte Gasphasenreaktion kann beispielsweise bei Temperaturen von 100 bis 500 ° C, vorzugsweise bei 150 bis 400 ° C und Drücken von 0,1 bis 50 bar, besonders bevorzugt bei 200 bis 300 ° C und Drücken von 0,5 bis 5 bar durchgeführt werden. Bei der Umsetzung in der Gasphase hält man vorteilhaft eine Katalysatorbelastung von 0,1 bis 20, insbesondere von 0,5 bis 8 g Ausgangsstoff der Formel II je g Katalysator und Stunde ein (WHSV = g Einsatzgemisch/g Katalysator und Stunde). Die Gasphasenreaktion kann in einem Festbett oder in einem Wirbelbett ausgeführt werden.

Nach der Umsetzung werden die entstandenen Produkte durch übliche Verfahren, z.B. durch Destillation aus dem Reaktionsgemisch isoliert; nichtumgesetzte Ausgangsstoffe werden gegebenenfalls in die Umsetzung zurückgeführt.

Bei dieser Arbeitsweise werden gasförmigen Reaktionsprodukte sofort in eine Trennung eingebracht und z.B. in einer Fraktionierkolonne in ihre Einzelkomponenten zerlegt. Eine bevorzugte Ausführungsform besteht darin, daß man die Reaktionsausträge in einer wäßrigen Hydrogencarbonat-Lösung z.B. $KHCO_3$ oder $NaHCO_3/Na_2SO_4$ quencht.

Die Substituenten $R^1$ bis $R^5$ in Formel I und II haben vorzugsweise folgende Bedeutung:

$R^1$, $R^2$ unabhängig voneinander

- Wasserstoff,
- unverzweigtes oder verzweigtes $C_1$- bis $C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- $C_3$- bis $C_8$-Cycloalkyl, wie Cyclopropyl, Cyclobutyl, Cyclopentyl, Cyclohexyl, Cycloheptyl und Cyclooctyl,
- Aryl, bevorzugt Phenyl, 1-Naphthyl und 2-Naphthyl, besonders bevorzugt Phenyl,
- ein- bis dreifach durch $C_1$- bis $C_4$-Alkyl substituiertes Aryl, wie 2-Methylphenyl, 3-Methylphenyl, 4-Methylphenyl, 2-Ethylphenyl, 3-Ethylphenyl, 4-Ethylphenyl, 2-n-Propylphenyl, 3-n-Propylphenyl, 4-n-Propylphenyl, 3-iso-Propylphenyl, 4-iso-Propylphenyl, 2-n-Butylphenyl, 3-n-Butylphenyl, 4-n-Butyl-phenyl, 3-iso-Butylphenyl, 4-iso-Butylphenyl, 3-sec.-Butylphenyl, 4-sec.-Butylphenyl, 3-tert.-Butylphenyl, 4-tert.-Butylphenyl, 2,4-Dimethylphenyl, 2,6-Dimethylphenyl, 3,5-Dimethylphenyl und 2,4,6-Trimethylphenyl,
- $C_7$- bis $C_{16}$-Aralkyl, bevorzugt $C_7$- bis $C_{10}$-Aralkyl wie Benzyl und Phenethyl,
- durch $C_1$- bis $C_4$-Alkyl ein- bis dreifach substituiertes $C_7$-bis $C_{16}$-Aralkyl, bevorzugt durch $C_1$- bis $C_4$-Alkyl ein- bis dreifach substituiertes $C_7$- bis $C_{10}$-Aralkyl wie 4-Methylbenzyl und 4-Methylphenethyl,

$R^3$

- $-CR^5(OR^4)_2$,
- $-COOR^4$,
- $-COR^4$,

$R^4$

- unverzweigtes oder verzweigtes $C_1$- bis $C_{20}$-Alkyl, bevorzugt unverzweigtes oder verzweigtes $C_1$- bis $C_{12}$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl,
- $C_7$- bis $C_{20}$-Alkylaryl, bevorzugt $C_7$- bis $C_{10}$-Alkylaryl wie 4-Methyl-phenyl und 4-Ethyl-phenyl,
- $C_7$- bis $C_{20}$-Aralkyl, bevorzugt $C_7$- bis $C_{10}$-Aralkyl wie Benzyl, 1-Phenyl-ethyl, 2-Phenyl-ethyl, 1-Phenyl-n-propyl, 2-Phenyl-n-propyl, 2-Phenyl-iso-propyl und 3-Phenyl-n-propyl,

$R^5$

- Wasserstoff
- $C_1$- bis $C_8$-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, tert.-Pentyl, neo-Pentyl, 1,2-Dimethylpropyl, n-Hexyl, iso-Hexyl, sec.-Hexyl, n-Heptyl, iso-Heptyl, n-Octyl und iso-Octyl.

Besonders bevorzugte Substituenten $R^1$ bis $R^5$ in Formel I und II haben folgende Bedeutung:

$R^1$, $R^2$

- unabhängig voneinander

- Wasserstoff
- $C_1$- bis $C_4$-Alkyl wie Methyl, Ethyl, n-Propyl, iso-Propyl, n-Butyl, iso-Butyl, sec.-Butyl und tert.-Butyl,

$R^3$

- $-CR^5(OR^4)_2$,
- $-COOR^4$,
- $-COR^4$,

$R^4$

- $C_1$- bis $C_2$-Alkyl wie Methyl und Ethyl

$R^5$

- Wasserstoff
- $C_1$- bis $C_2$-Alkyl wie Methyl und Ethyl.

Ausgangsstoffe der allgemeinen Formel II sind z.B.

1,1,2,2-Tetramethoxipropan
1,1,2,2-Tetraethoxipropan
1,1,2,2-Tetraisobutoxipropan
1,1,2,2-Tetra-n-butoxipropan
2,2,3,3-Tetramethoxibutan
2,2,3,3-Tetra-n-butoxi-butan
2,3,3,3-Tetramethoxipentan
2,2-Dimethoxibutan-3-on
2,2-Di-n-butoxibutan-3-on
3,3-Dimethoxihexan-4-on
2,2-Dimethoxipropionsäuremethylester
2,2-Diisobutoxipropionsäureisobutylester
2,2-Dimethoxibuttersäuremethylester
2,2-Dimethoxi-3-methylbuttersäuremethylester

Die Ausgangsstoffe sind bekannt oder nach allgemein bekannten Methoden durch Acetalisierung der entsprechenden Aldehyde herstellbar.

Als Katalysatoren für das erfindungsgemäße Verfahren werden zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2 (siehe Ullmanns Encyclopädie d. techn. Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend der Struktur werden Zeolithe in verschiedene Gruppen unterteilt (siehe Ullmanns Encyclopädie d. techn. Chemie, 4. Auflage, Bd. 24, S. 575 (1983)). So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Das das erfindungsgemäße Verfahren in Betracht kommende Katalysatoren sind Zeolithe aus der Mordenitgruppe oder engporige Zeolithe vom Erionit- bzw. Chabasit-Typ oder Zeolithe vom Faujasit-Typ, z.B. Y-, X- oder L-Zeolithe. Zu dieser Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et. al. Elsevier Scientific Publishing Comp. 1980, S. 203 und "Crystal Structures of Ultra-stable Faujasites" Advances in Chemistry Series Nr. 101, American Chemical Society, Washington, DC, S. 226 ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise Al-$(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 200 °C unter autogenem Druck hergestellt. Auch gehören hierzu die isotaktischen Zeolithe nach EP-A-34 727 und EP-A-46 504. Die erhaltenen Aluminosilikatzeolithe erhalten je nach Wahl der Einsatzstoffmengen ein $SiO_2/Al_2O_3$-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter Druck synthetisiert, indem man eine Vorverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP-A-34 727 und EP-A-46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchgeführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Zu den verwendbaren siliciumreichen Zeolithen ($SiO_2/Al_2O_3$>10) gehören auch die sog. ZSM-Typen, Ferrierit, Nu-1 und Silicalit®, ein Molekularsieb, ein sog. Silica Polymorph.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h (h = Stunden) getrocknet und bei 500 °C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcelluose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abtrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, g, Zn, Seltene Erdmetalle wie La, Ce, Pr, Nd, Fr, Yb und U eingesetzt.

Zweckmäßigerweise führt man die Dotierung so durch, daß man z.B. den verformten Zeolithenh in einem Steigrohr vorlegt und bei 20 bis 100 °C z.B. eine wäßrige oder ammoniakalische Lösung eines

Halogenids oder eines Nitrats der voranbeschriebenen Metalle überleitet. Ein derartiger Ionenaustausch kann z.B.f an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x 3 $H_2$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst. Mit dieser Lösung wird der verformte oder unverformte Zeolith eine gewisse Zeit, ca. 30 Minuten, getränkt. Die eventuell überstehende Lösung wird am Rotationsverdampfer von Wasser befreit. Danach wird der getränkte Zeolith bei ca. 150 °C getrocknet und bei ca. 550 °C calciniert. Dieser Tränkvorgang kann mehrmals hintereinander vorgenommen werden, um den gewünschten Metallgehalt einzustellen.

Auch ist es möglich, z.B. eine wäßrige $Ni(CO_3)_2$-Lösung oder ammoniakalische $Pd(NO_3)_2$-Lösung herzustellen und darin den reinen pulverförmigen Zeolithen bei 40 bis 100 °C unter Rühren ca. 24 h aufzuschlämmen. Nach Abfiltrieren, Trocknen bei ca. 150 °C und Calcinierung bei ca. 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei ca. 150 °C getrocknet und bei ca. 550 °C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80 °C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 bis 500 °C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen, des zeolithischen Materials wird dieses zweckmäßig, z.B. bei Temperaturen von 100 bis 160 °C, getrocknet und bei Temperaturen von im allgemeinen 450 bis 600 °C calciniert. Gemäß einer weiteren bevorzugten Ausführungsform für die Säurebehandlung wird das zeolithische Material nach einer Verformung mit Bindemittel bei erhöhter Temperatur, zweckmäßig bei Temperaturen von 50 bis 90 °C, vorzugsweise 60 bis 80 °C, über einen Zeitraum von 0,5 bis 5, vorzugsweise mit 12 bis 20 gew.%iger Salzsäure, behandelt. Anschließend wird das zeolithische Material im allgemeinen ausgewaschen und zweckmäßig, z.B. bei Temperaturen von 100 bis 160 °C, getrocknet und bei Temperaturen von im allgemeinen 450 bis 600 °C calciniert. Einer HF-Behandlung kann sich auch eine HCL-Behandlung anschließen.

Nach einer anderen Arbeitsweise lassen sich Zeolithe durch Aufbringen von Phosphorverbindungen, wie Trimethoxyphosphat, Trimethoxyphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110 °C getrocknet und bei 500 °C calciniert.

Weitere Katalysatoren für die Herstellung von den erfindungsgemäßen bifunktionellen Bausteinen sind Phosphate, insbesondere Aluminiumphosphate, Siliciumaluminiumphosphate, Siliciumeisenaluminiumphosphate, Cerphosphat, Zirkonphosphate, Borphosphate, Eisenphosphate, Strontiumphosphate und deren Gemische.

Als Aluminiumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate sind z.B. APO-5, APO-9,

APO-11, APO-12, APO-14, APO-21, APO-25, APO-31 und APO-33. Synthesen dieser Verbindungen sind in EP 132 708, US 4 310 40 und US 4 473 663 beschrieben.

Beispielsweise das $AlPO_4$-5 (APO-5) wird synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150 °C 20 bis 60 h unter autogenem Druck in einem Autoklaven umsetzt. Das abfiltrierte $AlPO_4$ wird getrocknet und bei 100 bis 160 °C und calciniert bei 450 bis 550 °C.

$AlPO_4$-9 (APO-9) wird ebenfalls aus Orthophosphorsäure und Pseudoboehmit aber in wäßriger DABCO-Lösung (1,4-Diazabicyclo-(2,2,2)-octan) bei ca. 200 °C unter autogenem Druck während 200 bis 400 h synthetisiert.

Die Synthse des $AlPO_4$-21 (APO-32) erfolgt aus Orthophosphorsäure und Pseudoboehmit in wäßriger Pyrrolidon-Lösung bei 150 bis 200 °C und autogenem Druck während 50 bis 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP 103 117, US 4 440 871 beschrieben. SAPO's werden hergestellt durch Kristallisation aus wäßriger Mischung bei 100 bis 250 °C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischung von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200 °C während 20 bis 200 h unter autogenem Druck in einem Rührautoklaven erhalten. Die Trocknung des abfiltrierten Pulvers erfolgt bei 110 bis 160 °C und die Calcination bei 450 bis 550 °C.

Als Phosphorkatalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3 \times H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60 °C/16 h getrocknet.

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 bis 650 °C, vorzugsweise 300 bis 550 °C, herstellen.

Cerphosphat für das erfindungsgemäße Verfahren wird z.B. durch Fällung aus 52 g $Ce(NO_3)_3 \times 6\ H_2O$ und 56 g $NaH_2PO_4 \times 2\ H_2O$ erhalten. Nach der Filtration wird das Material zu Strängen verformt, bei 120 °C getrocknet und bei 450 ° calciniert.

Phosphorsäure wird auf $SiO_2$-, $Al_2O_3$- oder Bims-Träger z.B. durch Auftränken oder Versprühen aufgebracht. Ein Phosphorsäure-haltiger Katalysator kann beispielsweise durch Auftränken von $H_3PO_4$- oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden, danach schließt sich eine Trocknung und meist eine Calcination an. Phosphorsäure kann auch in einer Imprägniermühle auf das Trägermaterial aufgesprüht werden.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die Enolether I kann man zur Herstellung von Aldehyden in an sich bekannter Weise durch Umsetzung mit Kohlenmonoxid und Wasserstoff erhalten. Für die Verwendung der Verbindungen I sind solche Verbindungen bevorzugt, die in den Resten $R^1$ bis $R^5$ keine ungesättigten nichtaromatischen C-C-Bindungen enthalten.

Sie lassen sich nach allgemein bekannten Methoden nach folgender Gleichung hydroformylieren:

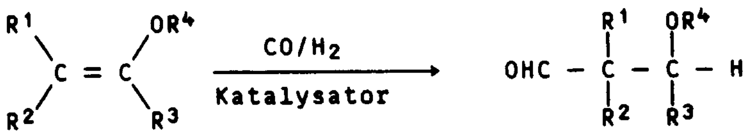


Als Katalysatoren eignen sich beispielsweise Rh-haltige Katalysatoren.

Die folgenden Beispiele veranschaulichen die Erfindung:

Herstellung der Katalysatoren

Katalysator A

Der Borosilikatzeolith des Pentasil-Typs wird in einer hydrothermalen Synthese aus 640 g hochdispersem $SiO_2$, 122 g $H_3BO_3$, 8000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew-% $SiO_2$ und 2,3 Gew.% $B_2O_3$.

Mit diesem Material werden durch Verformen mit einem Verformungshilfsmittel 2-mm-Stränge hergestellt, die bei 100 °C/16 h getrocknet und bei 500 °C/24 h calciniert werden.

Katalysator B

Ein Aluminosilikatzeolith vom Pentasil-Typ wurde unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wurde das kristalline Reaktionsprodukt bei 110 °C/24 h getrocknet und bei 500 °C/24 h calciniert. Dieser Aluminosilikatzeolith enthielt 91,6 Gew.% $SiO_2$ und 4,6 Gew.% $Al_2O_3$. Der Katalysator wurde mit einem Verformungshilfsmittel zu 2-mm-Stränge verformt, bei 110 °C/16 h getrocknet und bei 500 °C/24 h calciniert.

Katalysator C

Katalysator C wird erhalten, indem man die Stränge des Katalysators A mit einer wäßrigen $Cs_2CO_3$-Lösung imprägniert, danach bei 130 °C/2 h trocknet und bei 540 °C/2 h calciniert. Der CS-Gehalt beträgt 0,6 Gew.%.

Katalysator D

200 g des bei Katalysator A beschriebenen Borosilikatzeolith werden mit 1 l einer wäßrigen Lösung aus 16,7 g $FeCl_3$ x 6 $H_2O$ und 50 g $NH_4Cl$ 24 h bei Raumtemperatur ionenausgetauscht, danach gründlich mit $H_2O$ Cl-frei gewaschen, bei 15 °C/1 h getrocknet und bei 500 °C/2 h calciniert. Dieses Pulver wird mit hochdispersem $SiO_2$ im Gewichtsverhältnis 70:30 verformt. Nach Trocknung werden die Stränge bei 500 °C/16 h calciniert.

Katalysator E

Katalysator E wird wie Katalysator C hergestellt, wobei jedoch $Cs_2CO_3$ durch $Ce(NO_3)_2$ ersetzt wird. Der Ce-Gehalt beträgt 1,65 Gew.%.

Katalysator F

$AlPO_4$-12 (APO-12) wird synthetisiert, indem man 200 g 98 %ige Phosphorsäure und 136 g Boehmit in 400 g Wasser löst bzw. suspendiert, hierzu eine wäßrige Lösung aus 60 g Ethylendiamin und 320 g $H_2O$ zugibt und diese Mischung in einem Rührautoklaven bei 200 °C während 24 Std. unter autogenem Druck umsetzt. Nach Abfiltrieren wird das kristalline Material bei 120 °C getrocknet und bei 500 °C/16 h calciniert. Das so synthetisierte $AlPO_4$-12 enthält 55,5 Gew.% $P_2O_5$, 39,7 Gew.% $Al_2O_3$. Dieses Material wird mit Verstrangungshilfsmitteln zu 3-mm-Strängen verformt, abermals bei 120 °C getrocknet und bei °C/6 h calciniert.

Katalysator G

Siliciumaluminiumphosphat-5 (SAPO-5) wird hergestellt aus einer Mischung aus 200 g 98 %ige Phosphorsäure, 136 g Boehmit, 60 g Kieselsol (30 %ige) 287 g Tripropylamin und 587 g $H_2O$. Diese Mischung wird bei 150 °C während 168 Std. unter autogenem Druck umgesetzt. Nach Filtration wird das kristalline Produkt bei 120 °C getrocknet und bei 500 °C calciniert. SAPO-5 enthält 49,8 Gew.% $P_2O_5$ 33,0 Gew.% $Al_2O_3$, 6,2 Gew.% $SiO_2$. SAPO-5 wird mit einem Vertrangungshilfsmittel zu 3-mm-Strängen verformt, bei 120 °C getrocknet und bei 500 °C calciniert.

Katalysator H

Kommerziell erhältlich Zirkonphosphat $Zr_3(PO_4)_4$ wird verformt.

Katalysator I

$CePO_4$ wird durch Fällung aus 52 g $Ce(NO_3)_3$ x 6 $H_2O$ und 56 g $NaH_2PO_4$ x 2 $H_2O$ erhalten. Nach der Filtration wird das Material zu Strängen verformt, bei 120°C getrocknet und bei 450° Ccalciniert. Katalysator I enthält 47,1 Gew.% Ce und 12,7 Gew.% P.

Beispiele

Beispiel 1 bis 9

Die Herstellung der Enolether erfolgte nach folgender Methode

Die Reaktionen in der Gasphase wurden unter isothermen Bedingungen in einem Rohrreaktor (Wendel, 0,6 cm Innendurchmesser, 90 cm Länge) mindestens 6 Stunden lang durchgeführt. Die Reaktionsprodukte wurden durch übliche Methoden abgetrennt und charakterisiert. Es empfiehlt sich, das Reaktionsgas mit Methanol zu quenchen und die Lösung mit wäßriger Natriumbicarbonat zu neutralisieren, um Dimersation bzw. Rückreaktion zu verhindern. Die quantitative Bestimmung der Reaktionsprodukte und der Ausgangsprodukte erfolgte gaschromatographisch. Die GC-Analysen erfolgten nach 6 Stunden.

**Tabelle**

1.1.2.2-Tetramethoxypropan ------→ 1.1.2-Trimethoxyprop-2-en + Methanol

| Beispiel | 1 | 2 | 3 | 4 | 5 | 6 | 7 | 8 | 9 |
|---|---|---|---|---|---|---|---|---|---|
| Katalysator | A | B | C | D | E | F | G | H | I |
| Temperatur °C | 250 | 250 | 250 | 250 | 250 | 250 | 250 | 250 | 250 |
| WHSV $h^{-1}$ | 2 | 2 | 2 | 2 | 2 | 2 | 2 | 2 | 2 |
| Umsatz % (II) | 100 | 100 | 100 | 100 | 100 | 97 | 100 | 100 | 100 |
| Selektivität % (III) | 94,7 | 89,5 | 92,3 | 96,3 | 95,1 | 85,2 | 91,8 | 89,5 | 93,1 |

Herstellung von 1,1,2-Trimethoxyprop-2-en

Beispiel 10

Es werden 60 ml/h 1,1,2,2-Tetramethoxypropan (gelöst in THF 50:50 Gew.%) in einem Stickstoffstrom

von 200 l/h verdampft und bei einer Temperatur von 260 °C über 1 l eines Borzeolithkatalysators (Katalysator A), der in einem von außen elektrisch beheizten Reaktionsrohr untergebracht ist, geleitet.

Das gasförmige Reaktionsgemisch während einer 10stündigen Reaktionszeit wird kondensiert und in einer auf (-80) °C gekühlten Vorlage aufgefangen. Die Reinherstellung des 1,1,2-Trimethoxyprop-2-en (2-Methoxyacroleindimethylacetals) gelingt in einfacher Weise durch eine übliche Destillation.

Der Umsatz beträgt 98,9 %, die Selektivität 89,4 %.

Beispiel 11

Es wird wie in Beispiel 1 bis 9 verfahren, die Temperatur beträgt jedoch 270 °C und die Gesamtversuchsdauer 74 Std.

Das gasförmige Reaktionsgemisch wird in den Mittelteil einer Fraktionierkolonne geleitet, das gebildete Methanol sowie andere Leichtsieder destillieren über Kopf ab, während 1,1,2-Trimethoxyprop-2-en mit geringen Mengen nicht umgesetzten Ausgangsprodukt im Sumpf der Kolonne abgezogen werden können.

Die Reinherstellung gelingt in einfacher Weise durch eine übliche Destillation.

Der Umsatz von 1,1,2,2-Tetramethoxypropan ist >98 %, die Destillationsausbeute 87,2 %.

Beispiel 12

In der gleichen Apparatur und am gleichen Katalysator wie Beispiel 1 werden 120 ml/h 1,1,2,2-Tetramethoxypropan in einem Stickstoffstrom von 200 h/l verdampft und bei 230 °C umgesetzt. Die Reaktion wurde 20 h lang durchgeführt.

Das gasförmige Reaktionsgemisch wurde mit Methanol gequencht und in eine gesättigte wäßrige Natriumcarbonat-Lösung eingeleitet.

Nach üblicher Aufarbeitung werden 100 % Umsatz und 70,5 % Selektivität am 1,1,2-Trimethoxiprop-2-en erhalten.

Die folgenden Beispiele beschreiben die Verwendung der Enolether:

Herstellung von 3,4,4,-Tri-isobutoxybutanal

Beispiel 13

Herstellung von 3,4,4-Tri-isobutoxybutanal

In einem mit Magnetrührer ausgestatteten Mini-autoklaven (Reaktionsraum 300 ml) werden 71 g 2,3,3,-Tri-isobutoxy-prop-1-en in 70 g Tetrahydrofuran gelöst. Als Oxierungskatalysator werden 150 mg $Rh_2(COD)_2Cl_2$ (COD = Cyclo-octa-dien-1,5) zugegeben. Die Hydroformylierung wird bei 110 °C bei einem Synthesegasdruck ($CO:H_2$ = 1:1) von 300 bar vorgenommen. Im Verlauf von 20 Stunden werden 300 bar Synthesegas nachgepreßt. Das Reaktionsgemisch von 147 g wird durch fraktionierte Destillation aufgearbeitet. Im Temperaturbereich zwischen 118 - 136 °C gehen bei einem Druck von 2 mbar 80 g über. Nach der NMR-, der 13 C-NMR, und der IR-Spektren-analyse liegt das 3,4,4-Tri-isobutoxy-butanal vor.

Beispiel 14

In einem 10-l-Magnethubrührautoklaven werden 1 015 g 2,3,3-Trimethoxy-prop-1-en zusammen mit 3 000 g Tetrahydrofuran und als Hydroformylierungskatalysator 500 mg $Rh_2(COD)_2Cl_2$ eingefüllt. Die Oxierung wird unter einem Druck von 500 bar $CO/H_2$ = 1:1 bei 40 °C 6 Stunden, bei 60 °C/550 bar -6 Stunden und bei 80 °C/600 bar weitere 6 Stunden durchgeführt. Nach der gaschromatographischen Analyse wird bei dieser Umsetzungsart ein Umsatz von 94 % erzielt. Die gewünschte Verbindung 3,4,4-Trimethoxy-butanal (Kp = 135 °C bei 110 mbar) wurde nach der Fraktionierung in einer Ausbeute von 87 % erhalten.

Herstellung von 2-Methoxyacrylsäuremethylester

Beispiel 15

Es werden 100 ml/h 2,2-Dimethoxypropionsäuremethylester in einem Stickstoffstrom von 300 l/h verdampft und bei einer Temperatur von 230 °C über 1 l Katalysator A, der in einem von außen elektrisch beheizten Reaktionsrohr untergebracht ist, geleitet.

Das gasförmige Reaktionsgemisch wird während einer 10stündigen Reaktionsperiode kondensiert und in einer Pufferlösung mit ph = 7 gequencht.

Nach üblicher Aufarbeitung beträgt der Umsatz 100 % und die Selektivität des 2-Methoxyacrylsäuremethylester 94,5 %.

## Patentansprüche

**1.** Verfahren zur Herstellung von Enolethern der allgemeinen Formel I

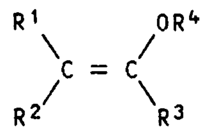

(I)

durch Abspaltung des Restes $R^4OH$ aus Acetalen oder Ketalen der Formel II

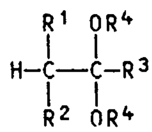

(II)

in denen die Substituenten

| | |
|---|---|
| $R^1$ und $R^2$ | unabhängig voneinander Wasserstoff, $C_1$- bis $C_{12}$-Alkyl, $C_3$- bis $C_8$-Cycloalkyl, $C_3$- bis Ca-Cycloalkenyl, gegebenenfalls durch $C_1$- bis $C_4$-Alkyl substituiertes Aryl oder $C_7$- bis $C_{16}$-Aralkyl, |
| $R^3$ | $-CR^5(OR^4)_2$, $-COOR^4$ oder $-COR^4$, |
| $R^4$ | $C_1$- bis $C_{20}$-Alkyl, $C_7$- bis $C_{20}$-Alkylaryl oder $C_7$- bis $C_{20}$-Aralkyl und |
| $R^5$ | Wasserstoff oder $C_1$- bis $C_8$-Alkyl bedeuten, |

dadurch gekennzeichnet, daß man die Umsetzung in Gegenwart von Phosphorsäure und/oder Hydrogenphosphaten auf einem Trägermaterial und/ oder Phosphaten und/oder Zeolithen als Katalysatoren vornimmt.

**2.** Verfahren nach Anspruch 1, dadurch gekennzeichnet, daß man die Umsetzung bei Temperaturen von 50 bis 500 °C vornimmt.

**3.** Verfahren nach den Ansprüchen 1 oder 2, dadurch gekennzeichnet, daß man die Umsetzung bei Drücken von 0,1 bis 50 bar vornimmt.

**4.** Verfahren nach den Ansprüchen 1 bis 3, dadurch gekennzeichnet, daß man die Umsetzung in der Gasphase gegebenenfalls unter Verwendung eines inerten Trägergases bei Temperaturen von 150 bis 400 °C und Drücken von 0,5 bis 5 bar vornimmt.

**5.** Verfahren nach den Ansprüchen 1 bis 3, dadurch gekennzeichnet, daß man die Umsetzung in einer Flüssigphasenreaktion gegebenenfalls in Gegenwart eines inerten Lösungsmittels bei Temperaturen von 50 bis 200 °C und Drücken von 0,5 bis 20 bar vornimmt.

**6.** Verfahren nach den Ansprüchen 1 bis 5, dadurch gekennzeichnet, daß Substituenten

| | |
|---|---|
| $R^1$, $R^2$ | unabhängig voneinander Wasserstoff oder $C_1$- bis $C_4$-Alkyl, |
| $R^3$ | $-CR^5(OR^4)_2$, $-COOR^4$ oder $-COR^4$, |
| $R^4$ | Methyl oder Ethyl, |
| $R^5$ | Wasserstoff, Methyl oder Ethyl bedeuten. |

**7.** Verfahren nach den Ansprüchen 1 bis 6, dadurch gekennzeichnet, daß man die Umsetzung in Gegenwart von Mono- und Dihydrogenphosphaten des Natriums auf Trägermaterialien oder Bor-, Aluminium-, Zirkon-, Eisen- oder Strontiumphosphaten oder deren Gemischen vornimmt.

8. Verfahren nach den Ansprüchen 1 bis 6, dadurch gekennzeichnet, daß man die Umsetzung in Gegenwart von Bor-, Aluminium-, Eisensilikatzeolithen oder mit Alkalimetallen, Übergangsmetallen, seltenen Erdmetallen dotierten Zeolithen oder Zeolithen des Pentasil- oder Faujasit-Typs oder deren Gemische vornimmt.

9. Verwendung der Enolether der Formel I nach Anspruch 1, in der
   - $R^1$, $R^2$ unabhängig voneinander Wasserstoff, $C_1$- bis $C_{12}$-Alkyl, $C_3$- bis $C_8$-Cycloalkyl, gegebenenfalls durch $C_1$- bis $C_4$-Alkyl substituiertes Aryl oder $C_7$- bis $C_{16}$-Aralkyl,
   - $R^3$ $-CR^5(OR^4)_2$, $-COOR^4$ oder $-COR^4$,
   - $R^4$ $C^1$- bis $C_{20}$-Alkyl, $C_7$- bis $C_{20}$-Alkylaryl oder $C_7$- bis $C_{20}$-Aralkyl,
   - $R^5$ Wasserstoff oder $C_1$- bis $C_8$-Alkyl,

   bedeuten, zur Herstellung von Aldehyden durch Umsetzung mit Kohlenmonoxid und Wasserstoff in an sich bekannter Weise.

## Claims

1. A process for preparing an enol ether of the general formula I

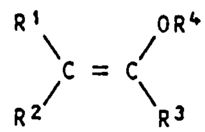

(I)

by eliminating $R^4OH$ from an acetal or ketal of the formula II

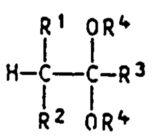

(II)

where
   - $R^1$ and $R^2$ are each independently of the other hydrogen, $C_1$-$C_{12}$-alkyl, $C_3$-$C_8$-cycloalkyl, $C_3$-$C_8$-cycloalkenyl, unsubstituted or $C_1$-$C_4$-alkyl-substituted aryl or $C_7$-$C_{16}$-aralkyl,
   - $R^3$ is $-CR^5(OR^4)_2$, $-COOR^4$ or $-COR^4$,
   - $R^4$ is $C_1$-$C_{20}$-alkyl, $C_7$-$C_{20}$-alkylaryl or $C_7$-$C_{20}$-aralkyl and
   - $R^5$ is hydrogen or $C_1$-$C_8$-alkyl,

   which comprises performing the reaction in the presence of phosphoric acid or a hydrogen phosphate on a carrier material or a phosphate or a zeolite as catalyst.

2. A process as claimed in claim 1, wherein the reaction is carried out at from 50 to 500 ° C.

3. A process as claimed in claim 1 or 2, wherein the reaction is carried out under from 0.1 to 50 bar.

4. A process as claimed in any of claims 1 to 3, wherein the reaction is carried out in the gas phase with or without an inert carrier gas at from 150 to 400 ° C under from 0.5 to 5 bar.

5. A process as claimed in any of claims 1 to 3, wherein the reaction is carried out in the liquid phase in the presence or absence of an inert solvent at from 50 to 200 ° C under from 0.5 to 20 bar.

6. A process as claimed in any of claims 1 to 5, wherein the substituents
   - $R^1$ and $R^2$ are each independently of the other hydrogen or $C_1$-$C_4$-alkyl,
   - $R^3$ is $-CR^5(OR^4)_2$, $-COOR^4$ or $-COR^4$,
   - $R^4$ is methyl or ethyl,
   - $R^5$ is hydrogen, methyl or ethyl.

7. A process as claimed in any of claims 1 to 6, wherein the reaction is carried out in the presence of a mono- or dihydrogenphosphate of sodium on a carrier material or a boron, aluminum, zirconium, iron or strontium phosphate or a mixture thereof.

8. A process as claimed in any of claims 1 to 6, wherein the reaction is carried out in the presence of a borosilicate, aluminosilicate or iron silicate zeolite or a zeolite doped with an alkali metal, a transition metal or a rare earth metal, or a zeolite of the pentasil or faujasite type or a mixture thereof.

9. Use of an enol ether of the formula I as claimed in claim 1 where
   $R^1$ and $R^2$ are each independently of the other hydrogen, $C_1$-$C_{12}$-alkyl, $C_3$-$C_8$-cycloalkyl, unsubstituted or $C_1$-$C_4$-alkyl-substituted aryl or $C_7$-$C_{16}$-aralkyl,
   $R^3$ is $-CR^5(OR^4)_2$, $-COOR^4$ or $-COR^4$,
   $R^4$ is $C_1$-$C_{20}$-alkyl, $C_7$-$C_{20}$-alkylaryl or $C_7$-$C_{20}$-aralkyl and
   $R^5$ is hydrogen or $C_1$-$C_8$-alkyl,
   for preparing an aldehyde by reacting the enol ether with carbon monoxide and hydrogen in a conventional manner.

**Revendications**

1. Procédé de préparation d'éthers énoliques de la formule générale

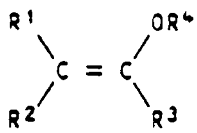

(I)

par séparation du reste $R^4OH$ d'acétals ou de cétals de la formule

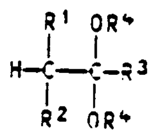

(II)

dans laquelle les symboles
   $R^1$ et $R^2$ représentent, indépendamment l'un de l'autre, des atomes d'hydrogène, des radicaux alkyle en $C_1$-$C_{12}$, cycloalkyle en $C_3$-$C_8$, cycloalcényle en $C_3$-$C_8$, aralkyle en $C_7$-$C_{16}$, ou aryle éventuellement substitué par des radicaux alkyle en $C_1$ à $C_4$,
   $R^3$ représente $-CR^5(OR^4)_2$, $-COOR^4$ ou $-COR^4$,
   $R^4$ représente un radical alkyle en $C_1$-$C_{20}$, alkylaryle en $C_7$ à $C_{20}$ ou aralkyle en $C_7$ à $C_{20}$ et
   $R^5$ représente un atome d'hydrogène ou un radical alkyle en $C_1$-$C_8$,
   caractérisé en ce que l'on entreprend la réaction en présence d'acide phosphorique et/ou d'hydrogénophosphates sur un support et/ou des phosphates et/ou des zéolithes servant de catalyseurs.

2. Procédé suivant la revendication 1, caractérisé en ce que la réaction s'entreprend à des températures de 50 à 500 °C.

3. Procédé suivant les revendications 1 ou 2, caractérisé en ce que l'on entreprend la réaction à des pression de 0,1 à 50 bars.

4. Procédé suivant les revendications 1 à 3, caractérisé en ce que l'on entreprend la réaction en phase gazeuse et en recourant éventuellement à l'emploi d'un gaz de support inerte à des températures de 150 à 400 °C et à des pressions de 0,5 à 5 bars.

5. Procédé suivant les revendications 1 à 3, caractérisé en ce que l'on entreprend la réaction sous forme

de réaction en phase liquide, éventuellement en présence d'un solvant inerte, à des températures de 50 à 200 °C et à des pressions de 0,5 à 20 bars.

**6.** Procédé suivant les revendications 1 à 5, caractérisé en ce que les symboles
$R^1$, $R^2$, représentent, indépendamment l'un de l'autre, des atomes d'hydrogène, ou des radicaux alkyle en $C_1$-$C_4$,
$R^3$ représente $-CR^5(OR^4)_2$, $-COOR^4$ ou $-COR^4$,
$R^4$ représente le radical méthyle ou éthyle,
$R^5$ représente un atome d'hydrogène, le radical méthyle ou le radical éthyle.

**7.** Procédé suivant les revendications 1 à 6, caractérisé en ce que l'on entreprend la réaction en présence de mono-et dihydrogénophosphates du sodium sur des supports ou des phosphates de bore, d'aluminium, de zirconium, de fer, ou de strontium, ou de leurs mélanges.

**8.** Procédé suivant les revendications 1 à 6, caractérisé en ce que l'on entreprend la réaction en présence de zéolithes de silicates de bore, d'aluminium, de fer, ou avec des zéolithes dopées de métaux alcalins, de métaux de transition, de métaux des terres rares ou des zéolithes du type pentasile ou faujasite, ou de leurs mélanges.

**9.** Utilisation des éthers énoliques de la formule 1 selon la revendication 1, dans laquelle
$R^1$ et $R^2$ représentent, indépendamment l'un de l'autre, des atomes d'hydrogène, des radicaux alkyle en $C_1$-$C_{12}$, cycloalkyle en $C_3$-$C_8$, cycloalcényle en $C_3$-$C_8$, aralkyle en $C_7$-$C_{16}$, ou aryle éventuellement substitués par des radicaux alkyle en $C_1$ à $C_4$,
$R^3$ représente $-CR^5$ $(OR^4)_2$, $-COOR^4$ ou $-COR^4$,
$R^4$ représente un radical alkyle en $C_1$-$C_{20}$, alkylaryle en $C_7$ à $C_{20}$ ou aralkyle en $C_7$ à $C_{20}$ et
$R^5$ représente un atome d'hydrogène ou un radical alkyle en $C_1$-$C_8$,
pour la préparation d'aldéhydes par réaction avec le monoxyde de carbone et l'hydrogène, de manière en soi connue.